(19) Europäisches Patentamt
European Patent Office
Office européen des brevets

(11) EP 3 108 871 A1

(12) **EUROPEAN PATENT APPLICATION**

(43) Date of publication:
**28.12.2016 Bulletin 2016/52**

(21) Application number: **15173588.3**

(22) Date of filing: **24.06.2015**

(51) Int Cl.:
***A61K 8/72*** *(2006.01)* ***C07G 1/00*** *(2011.01)*
***C08H 7/00*** *(2011.01)* ***C08L 97/00*** *(2006.01)*
***A61Q 5/02*** *(2006.01)* ***A61Q 5/12*** *(2006.01)*
***A61Q 19/00*** *(2006.01)* ***C11D 3/382*** *(2006.01)*
***C09G 1/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Procter & Gamble International Operations SA**
**1213 Geneva (CH)**

(72) Inventors:
- **BROOKER, Anju Deepali Massey**
**Newcastle upon Tyne, Tyne and Wear NE12 9TS (GB)**
- **VACCARO, Mauro**
**Newcastle upon Tyne, Northumberland NE12 9TS (GB)**
- **SCIALLA, Stefano**
**0040 Pomezia (IT)**
- **CRESTINI, Claudia**
**00173 Rome (IT)**
- **LANGE, Heiko**
**00173 Rome (IT)**

(74) Representative: **Howard, Phillip Jan**
**Procter & Gamble**
**Technical Centres Limited**
**Whitley Road**
**Longbenton**
**Newcastle upon Tyne NE12 9TS (GB)**

(54) **CONSUMER GOODS PRODUCT COMPRISING CARBOXYLATED LIGNIN OLIGOMER**

(57) The present invention relates to a consumer goods product comprising a consumer goods product ingredient and a non-cross linked functionalised lignin oligomer, wherein the lignin oligomer: (a) has a number average molecular weight ($\overline{M}_n$) in the range of from 800 Da to 1,800 Da; (b) comprises the functional group:

lignin_backbone-O-L-COOH

wherein: 'lignin_backbone' is the lignin structural backbone; and L is a linker comprising an - OH moiety; (c) has a hydroxyl content of at least 3 mmol/g; and (d) has a functionalisation content of from 0.2 mmol/g to 2.3 mmol/g.

EP 3 108 871 A1

## Description

FIELD OF THE INVENTION

The present invention relates to consumer goods products comprising carboxylated lignin oligomer.

BACKGROUND OF THE INVENTION

Carboxylated lignins provide anti-oxidant benefits and can act as a surface deposition aid in consumer goods products, such as skin treatment compositions, hair treatment compositions, oral care compositions home care compositions and detergent compositions (especially hand wash detergents). In addition, for home care applications, lignins can also provide surface modification benefits which lead to improved shine and water sheeting benefits.
However, the carboxylation of lignin depletes the hydroxyl content of lignin. Typically, the carboxy containing moiety used to functionize the lignin reacts with a hydroxyl group present on the lignin, forming an ether link. This loss of hydroxyl content of the functionalised lignin limits the extent of the solubility and surface affinity improvement observed by the carboxylisation. Depletion of the hydroxyl content of the lignin lowers its solubility and surface affinity.
The inventors have found that ensuring that the carboxy containing moiety additionally comprises a hydroxyl moiety, preserves the hydroxy content of the carboxylated lignin, and provides a carboxylated lignin oligomer having further improved solubility and surface affinity.

SUMMARY OF THE INVENTION

The present invention relates to a consumer goods product comprising a consumer goods product ingredient and a non-cross linked functionalised lignin oligomer, wherein the lignin oligomer: (a) has a number average molecular weight ($\overline{M}_n$) in the range of from 800 Da to 1,800 Da; (b) comprises the functional group:

lignin_backbone-O-L-COOH
wherein: 'lignin_backbone' is the lignin structural backbone; and L is a linker comprising an-OH moiety; (c) has a hydroxyl content of at least 3 mmol/g; and (d) has a functionalisation content of from 0.2 mmol/g to 2.3 mmol/g.

DETAILED DESCRIPTION OF THE INVENTION

**Consumer goods product:** The consumer goods product comprises a consumer goods product ingredient and a non-cross linked functionalised lignin oligomer.
The consumer goods product may comprise an emollient and/or humectant.
The consumer goods product may comprise an emulsifier, this may be preferred when the lignin oligomer is in the form of an emulsion.
The consumer goods product may be a skin treatment composition.
The consumer goods product may be a hair treatment composition.
The consumer goods product may be an oral care composition.
The consumer goods product may be an antiseptic cream.
The consumer goods product may be a shoe polish.
The consumer goods product may be a detergent composition.
The consumer goods product may comprise chitin and/or chitin derivatives.
The consumer goods product is typically selected from: feminine pad; diaper; razor blade strip; hard surface cleaning sheet and/or wipe; and teeth treatment strip. The consumer goods product is typically selected from: skin cream; skin lotion; shaving preparation gel or foam; handwash laundry detergent; handwash dishwashing detergent; soap bar; liquid handwash soap; body wash; toothpaste; shampoo; and conditioner.
**Consumer goods product ingredient:** Suitable consumer goods product ingredients include emmolient, humectants, emulsifiers, and any combination thereof.
**Non-cross linked functionalised lignin oligomer:** The lignin oligomer is non-cross linked and: (a) has a number average molecular weight ($\overline{M}_n$) in the range of from 800 Da to 1,800 Da; (b) comprises the functional group:

lignin_backbone-O-L-COOH
wherein: 'lignin_backbone' is the lignin structural backbone; and L is a linker comprising an-OH moiety; (c) has a hydroxyl content of at least 3 mmol/g; and (d) has a functionalisation content of from 0.2 mmol/g to 2.3 mmol/g.

Preferably, the lignin oligomer has a functionalisation content of from 0.6 mmol/g to 1.0 mmol/g.

Preferably, the lignin oligomer has a hydroxyl content of from 3 mmol/g to 5.7 mmol/g. Preferably, the lignin oligomer comprises less than 1wt% sulphur content.

Preferably, the lignin oligomer has a molar ratio of aromatic hydroxyl content to aliphatic hydroxyl content in the range of from 1:1 to 1.5:1.

Preferably, the lignin oligomer has a weight average molecular weight ($M_w$) in the range of from 800 Da to 5000 Da.

Preferably, the lignin oligomer has a number average molecular weight ($\overline{M}_n$) in the range of from 800 Da to 1200 Da.

Preferably, the lignin oligomer is essentially free of sulphur.

Preferably, the lignin oligomer has an ester content in the range of from 0.0 mmol/g to 0.1 mmol/g.

Preferably, the lignin oligomer is derived from corn, sugar cane, wheat and any combination thereof

Preferably, the lignin oligomer is obtained by an organosolv-like isolation process for the lignins, using preferentially wheat straw, corn stover and/or sugar cane bagasse lignin starting materials.

Preferably, the ratio of aromatic hydroxyl groups to aliphatic hydroxyl groups of the lignin oligomer is within the range of 1.2 to 1.9.

Preferably, the lignin oligomer has a hydrolysable ester content in the range of from 0.2 to 0.5 mmol/g. The hydrolysable ester content preferably comprises acetate and formate functional groups.

**Functional group:** The functional group has the the structure:

lignin_backbone-O-L-COOH

**Linker (L):** The linker (L) typically has a chemical structure:

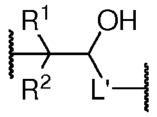

and / or

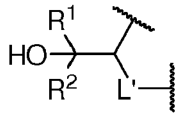

wherein R1 and R2 are independently chosen from a group consisting of H and linear or branched, saturated or unsaturated, substituted or unsubstituted $C_1$ to $C_{18}$ alkyl; and wherein L' is a linking motif chosen from linear or branched, saturated or unsaturated, substituted or unsubstituted $C_1$-$C_{18}$ alkyl.

The structural motifs (for L' formula) shown above are obtained preferentially, but not exclusively via reaction of activated hydroxyl groups directly being a part of the structural features making up the lignin backbone with reactive species carrying preferentially but not exclusively an epoxide functionality or a hydroxyl group on an aliphatic chain with a leaving group in α-position; this leaving group is preferentially, but not exclusively chosen from the group of chloride, bromide, iodide, mesylate, triflate, tosylate.

**Functionalisation of lignin with carboxylic groups:** Lignin (500 mg) is dissolved in water containing sodium hydroxide (amount corresponding to 1 equivalnt (eq.) to total acidic groups in the lignin, *i.e.,* phenolic hydroxyl and carboxylic acid groups). After 1 h of stirring, the epoxide-terminated carboxylic acid functional is added (depending on the desired technical loading, e.g. in range of from 0.25 to 10.0 eq. to lignin phenolic hydroxyl groups) and the reaction mixture is stirred at 50 °C overnight. In order to assure appropriate mixing of lignin and functional in the reaction mixture, additives such as emulsifiers, e.g., non-ionic surfactants, can be used.

After cooling to room temperature and acidifying to pH 2 using 10% (v/v) aqueous hydrogen chloride solution, the resulting suspension is centrifuged (15 min at 500 rpm) to recover the precipitated lignin. The functionalised lignin is then washed 3 times with 50 mL acidified water (pH 2) followed by renewed isolation *via* centrifugation (15 min at 500 rpm) each time. The final pellet was subsequently freeze-dried. The freeze-dried material is used for analysis and application without any additional manipulation.

**Measurement of the carboxyl functionalisation content:** The determination of the technical loading of a given carboxylated lignin with a given added functional is determined as follows: Ca. 30 mg of the carboxylated lignin are accurately weighed in a volumetric flask and suspended in 400 μL of the above prepared solvent solution. One hundred microliters of the internal standard solution are added, followed by 100 μL of 2-chloro-4,4,5,5-tetramethyl-1,3,2-dioxaphospholane (Cl-TMDP). The flask is tightly closed, and the mixture is stirred for 120 min at ambient temperature. $^{31}P$ NMR spectra are recorded using suitable equipment under the conditions reported above for the determination of aliphatic and aromatic hydroxyl contents. Quantitative analysis is done according to the procedure outlined above for the determination of aliphatic and aromatic hydroxyl contents, as also illustrated shown in Table 1.

Technical loadings are determined by comparing the abundancies of total aromatic hydroxyl groups of the

product lignin with the starting lignin, corrected for background hydrolysis reactions.

**Method of measuring sulphur content:** The chemical composition of a lignin sample in terms of its carbon (C), hydrogen (H), nitrogen (N) and sulphur (S) content can be determined by elemental analysis in form of a CHNS analysis of at least three different representative samples of a given batch of the respective lignin. Typical sample sizes are 2.0 mg of a lignin sample that was oven-dried at 105°C until a steady weight was obtained. The samples are placed in aluminum dishes and analyzed using a Carlo-Erba NA 1500 analyzer, using helium as carrier gas. Carbon (C), hydrogen (H), nitrogen (N) and sulphur (S) were detected in form of carbon dioxide, water, nitrogen, and sulphur dioxide, which are chromatographically separated to exit the instrument in the order of nitrogen, carbon dioxide, water, and sulphur dioxide. Quantification is achieved against calibrations using typical standard substances used for the calibration of elemental analysers, such as (bis(5-tert-butyl-2-benzo-oxazol-2-yl) thiophene, based on the peak areas of the chromatograms obtained for each lignin sample.

**Method of measuring $\overline{M}_n$ and $\overline{M}_w$:** The number average molecular weight, $\overline{M}_n$, as well as the weight average molecular weight, $\overline{M}_w$, can be determined using gel permeation chromatography (GPC). Prior to analysis, representative lignin samples are acetobrominated as reported in archival literature (J. Asikkala, T. Tamminen, D. S. Argyropoulos, J. Agric. Food Chem. 2012, 60, 8968-8973.) to ensure complete solubilisation in tetrahydrofuran (THF). 5mg lignin is suspended in 1mL glacial acetic acid/acetyl bromide (9:1 v/v) for 2 h. The solvent is then removed under reduced pressure, and the residue is dissolved in HPLC-grade THF and filtered over a 0.45 μm syringe filter prior to injection into a 20 μL sample loop. Typical analysis set-ups resemble the following specific example: GPC-analyses are performed using a Shimadzu instrument consisting of a controller unit (CBM-20A), a pumping unit (LC 20AT), a degasser unit (DGU-20A3), a column oven (CTO-20AC), a diode array detector (SPD-M20A), and a refractive index detector (RID-10A); the instrumental set-up is controlled using the Shimadzu LabSolution software package (Version 5.42 SP3). Three analytical GPC columns (each 7.5 x 30 mm) are connected in series for analyses: Agilent PLgel 5 μm 10000 Å, followed by Agilent PLgel 5 μm 1000 Å and Agilent PLgel 5 μm 500 Å. HPLC-grade THF (Chromasolv®, Sigma-Aldrich) is used as eluent (isocratic at 0.75 mL $min^{-1}$, at 40 °C). Standard calibration is performed with polystyrene standards (Sigma Aldrich, MW range 162 - 5 x 106 g $mol^{-1}$), and lower calibration limits are verified / adjusted by the use of synthesized dimeric and trimeric lignin models. Final analyses of each sample is performed using the intensities of the UV signal at λ = 280 nm employing a tailor-made MS Excel-based table calculation, in which the number average molecular weight ($\overline{M}_n$) and the weight average molecular weight ($\overline{M}_w$)) is calculated based on the measured absorption (in a.u.) at a given time (min) after corrections for baseline drift and THF-stemming artifacts.

$\overline{M}_n$ is calculated according to the formula

$$\overline{M}_n = \frac{\sum w_i}{\sum \frac{w_i}{M_i}}$$

in which $\overline{M}_n$ is the number average molecular weight

$w_i$ is obtained via $w_i = -h_i \frac{dV}{d(\log M)}$ with M being molecular weight hi being the signal intensity of a given logM measurement point

V being the volume of the curve over a given logM interval d(logM).

$M_i$ is a given molecular weight.

The analysis is run in triplicate, and final values are obtained as the standard average.

$\overline{M}_w$ is calculated according to the formula

$$\overline{M}_w = \frac{\sum w_i M_i}{\sum w_i}$$

in which $\overline{M}_w$ is the number average molecular weight

$w_i$ is obtained via $w_i = -h_i \frac{dV}{d(\log M)}$ $w_i$ with M being the molecular weight hi being the signal intensity of a given logM measurement point

V being the volume of the curve over a given logM interval d(logM).

$M_i$ is a given molecular weight.

The analysis is run in triplicate, and final values are obtained as the standard average.

Eventually necessary adjustment of $\overline{M}_n$ and $\overline{M}_w$ with respect to the desired applications is achieved by mechanical breaking of polymeric lignin using a ball mill, by chemically or enzymatically polymerising oligomeric lignin.

**Method of measuring aromatic hydroxyl and aliphatic hydroxyl content:** Typically, a procedure similar to the one originally published can be used (A. Granata, D. S. Argyropoulos, J. Agric. Food Chem. 1995, 43, 1538-1544). A solvent mixture of pyridine and (CDCl3) (1.6:1 v/v) is prepared under anhydrous conditions. The NMR solvent mixture is stored over molecular sieves (4 Å) under an argon atmosphere. Cholesterol is used as internal standard at a concentration of 0.1 mol/L in the aforementioned NMR solvent mixture. 50 mg of Cr(III) acetyl acetonate are added as relaxation agent to this standard solution.

Ca. 30 mg of the lignin are accurately weighed in a volumetric flask and suspended in 400 µL of the above prepared solvent solution. One hundred microliters of the internal standard solution are added, followed by 100 µL of 2-chloro-4,4,5,5-tetramethyl-1,3,2-dioxaphospholane (Cl-TMDP). The flask is tightly closed, and the mixture is stirred for 120 min at ambient temperature. 31P NMR spectra are recorded using suitable equipment, similar or identical to the following example: On a Bruker 300 MHz NMR spectrometer, the probe temperature is set to 20 °C. To eliminate NOE effects, the inverse gated decoupling technique is used. Typical spectral parameters for quantitative studies are as follows: 90° pulse width and sweep width of 6600 Hz. The spectra are accumulated with a delay of 15 s between successive pulses. Line broadening of 4 Hz is applied, and a drift correction is performed prior to Fourier transform. Chemical shifts are expressed in parts per million from 85 % H3PO4 as an external reference. All chemical shifts reported are relative to the reaction product of water with Cl-TMDP, which has been observed to give a sharp signal in pyridine/CDCl3 at 132.2 ppm. To obtain a good resolution of the spectra, a total of 256 scans are acquired. The maximum standard deviation of the reported data is 0.02 mmol/g, while the maximum standard error is 0.01 mmol/g. (A. Granata, D. S. Argyropoulos, J. Agric. Food Chem. 1995, 43, 1538-1544). Quantification on the basis of the signal areas at the characteristic shift regions (in ppm, as reported in A. Granata, D. S. Argyropoulos, J. Agric. Food Chem. 1995, 43, 1538-1544) is done using a tailor-made table calculation in which the abundances, given in mmol/g, of the different delineable phosphitylated hydroxyl groups are determined on the basis of the integral obtained for the signal of the internal standard, that is present in the analysis sample at a concentration of 0.1 m, creating a signal at the interval ranging from 144.5 ppm to 145.3 ppm. The area underneath the peak related to the internal standard is set to a value of 1.0 during peak integration within the standard processing of the crude NMR data, allowing for determining abundances using simple rule-of-proportion mathematics under consideration of the accurate weight of the sample used for this analysis. The analysis is run in triplicate, and final values are obtained as the standard average.

**Method of measuring hydrolysable ester content:** The total ester content of the lignin can be determined by subjecting the lignin to alkaline hydrolysis conditions: Ca. 500 mg of lignin are dissolved in an excess of 1 M sodium hydroxide solution and heated to temperatures of above 70 - 80 °C for 12 h. The lignin is subsequently precipitated by acidifying the reaction mixture, isolated and freeze-dried.

Ca. 30 mg of the lignin are accurately weighed in a volumetric flask and suspended in 400 µL of the above prepared solvent solution. One hundred microliters of the internal standard solution are added, followed by 100 µL of 2-chloro-4,4,5,5-tetramethyl-1,3,2-dioxaphospholane (Cl-TMDP). The flask is tightly closed, and the mixture is stirred for 120 min at ambient temperature. $^{31}$P NMR spectra are recorded using suitable equipment under the conditions reported above for the determination of aliphatic and aromatic hydroxyl contents. Quantification of the acid content is done on the basis of the signal intensities at the characteristic shift regions (in ppm) using a tailor-made table calculation referring to the signal of the internal standard. Abundances are typically given in mmol/g. The ester content is obtained as the difference in the abundances of acid groups, aliphatic hydroxyl groups, and aromatic hydroxyl groups found in untreated vs. the lignin treated with sodium hydroxide as outlined above. The analysis is run in triplicate, and final values are obtained as the standard average.

**Emollient:** Suitable emollients are silicon based emollients. Silicone-based emollients are organo-silicone based polymers with repeating siloxane (Si 0) units. Silicone-based emollients of the present invention are hydrophobic and exist in a wide range of molecular weights. They include linear, cyclic and crosslinked varieties. Silicone oils are generally chemically inert and usually have a high flash point. Due to their low surface tension, silicone oils are easily spreadable and have high surface activity. Examples of silicon oil include: Cyclomethicones, Dimethicones, Phenyl-modified silicones, Alkyl-modified silicones, Silicones resins, Silica. Other emollients useful in the present invention can be unsaturated esters or fatty esters. Examples of unsaturated esters or fatty esters of the present invention include: Caprylic Capric Triglycerides in combination with Bis-PEG/PPG-1 6/16 PEG/PPG-16/16 Dimethicone and C12-C15 Alkylbenzoate.

The basic reference of the evaluation of surface tension, polarity, viscosity and spreadability of emollient can be found under Dietz, T., Basic properties of cosmetic oils and their relevance to emulsion preparations. SOFW-Joumal, July 1999, pages 1-7.

**Humectant:** A humectant is a hygroscopic substance used to keep things moist. Typically, it is often a molecule

with several hydrophilic groups, most often hydroxyl groups; however, amines and carboxyl groups, sometimes esterified, can be encountered as well (its affinity to form hydrogen bonds with molecules of water is the crucial trait). A humectant typically attracts and retains the moisture in the air nearby via absorption, drawing the water vapour into and/or beneath the organism/object's surface.

Suitable humectants include: Propylene glycol, hexylene glycol, and butylene glycol, Glyceryl triacetate, Neoagarobiose, Sugar alcohols (sugar polyols) such as glycerol, sorbitol, xylitol, maltitol, Polymeric polyols such as polydextrose, Quillaia, Urea, Aloe vera gel, MP diol, Alpha hydroxy acids such as lactic acid, Honey, Lithium chloride

**Emulsifier:**_An emulsifier generally helps disperse and suspend a discontinuous phase within a continuous phase in an oil-in-water emulsion. A wide variety of conventional emulsifiers are suitable for use herein. Suitable emulsifiers include: hydrophobically-modified cross-linked polyacrylate polymers and copolymers, polyacrylamide polymers and copolymers, and polyacryloyldimethyl taurates. More preferred examples of the emulsifiers include: acrylates/C10-30 alkyl acrylate cross-polymer having tradenames PemulenTM TR-1, PemulenTM TR-2 (all available from Lubrizol); acrylates/steareth-20 methacrylate copolymer with tradename ACRYSOLTM 22 (from Rohm and Hass); polyacrylamide with tradename SEPIGEL 305 (from Seppic).

**Example 1:**

**Sample A (hydroxyl-neutral functionalisation):** Carboxyl functionalised lignin oligomer in accordance with the present invention, wherein the hydroxyl content of the lignin oligomer is preserved during the carboxylation reaction due to the presence of a hydroxyl moiety in the linker (L).

**Sample B (hydroxyl-consuming functionalisation):** Carboxyl functionalised lignin oligomer (comparative example), wherein the hydroxyl content of the lignin oligomer is depleted during the carboxylation reaction (no hydroxyl moiety present in the linker (L).

The chemical structures of samples A and B are shown below.

**hydroxyl-neutral functionalisation**

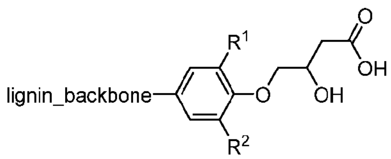

and / or

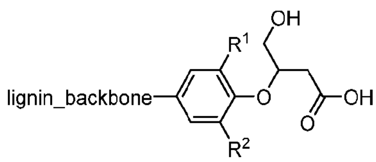

**H-type**: $R^1 = R^2 = H$

**G-type**: $R^1 = H$, $R^2 = OMe$

**S-type**: $R^1 = R^2 = OMe$

and / or

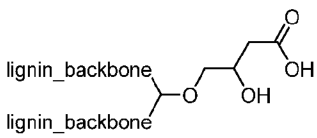

and / or

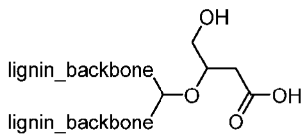

and / or

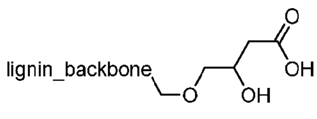

and / or

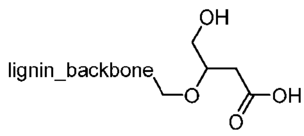

**hydroxyl-consuming functionalisation**

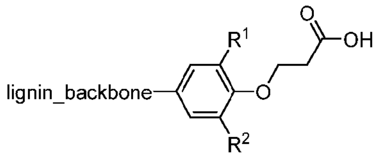

**H-type**: $R^1 = R^2 = H$

**G-type**: $R^1 = H$, $R^2 = OMe$

**S-type**: $R^1 = R^2 = OMe$

and / or

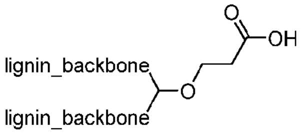

and / or

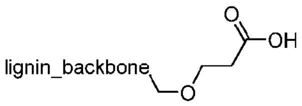

**Properties of lignin samples:**

| lignin | Carboxyl functionalisation content [a] | Mn [b] (Da) | $OH_{ali.}$ [c] (mmol/g) | $OH_{arom.}$ [c] (mmol/g) | total OH [c] (mmol/g) |
|---|---|---|---|---|---|
| **Sample A lignin oligomer [e]** | 0.75 | 1000-1100 | 1.85 | 1.45 | 3.30 |
| **Sample B lignin oligomer [f]** | 0.6 | 1000-1100 | 1.25 | 1.60 | 2.85 |

a: Determined *via* comparative quantitative $^{31}P$ nuclear magnetic resonance spectroscopy of phosphitylated sample.
b: Determined *via* gel permeation chromatography of acetylated/acetobrominated samples in THF.
c: Determined via $^{31}P$ nuclear magnetic resonance spectroscopy of phosphitylated sample.
e: Wheat straw lignin functionalised in 'hydroxyl-group-neutral' fashion.
f: Wheat straw lignin functionalised in a 'hydroxyl-consuming' fashion.

**Preparation of Turbidity Samples:** Weigh out 0.1g of functionalised lignin oligomer and dispersed in 1 litre of deionized water and stir it for 15 minutes at 200 rpm at room temperature. Then, measure the turbidity of the aqueous dispersion using the above method with Turbiscan Ageing Station system. Using sodium carbonate, pH was increased by one unit increments and turbidity was measured at pH 8.

**Turbidity Data:**

| Sample | % Transmission |
|---|---|
| Sample A: carboxyl functionalized lignin oligomer (hydroxyl neutral) | 74.80 |
| Sample B: carboxyl functionalized lignin oligomer (hydroxyl consuming) | 9.60 |

Sample A in accordance with the present invention showed superior solubility properties than the comparison example (Sample B).

**Process of making the samples:** Preparation of contact angle samples: Weigh out 0.1g of functionalised lignin oligomer and disperse in 1 litre of deionized water dispersion and stir it for 15 minutes at 200 rpm at room temperature. Using sodium carbonate, pH was adjusted to pH 10.5. Then, glass slides were dipped for 30 minutes and allowed to dry two hours at room temperature. Following this preparatory procedure contact angle of deionized water on the treated surface was measured using First Ten Angstroms 200 equipment.

**Contact Angle Data:**

| | Δ Contact Angle |
|---|---|
| Sample A: carboxyl functionalised lignin (hydroxyl neutral) | -6.3° |
| Sample B: lignin functionalised with carboxyl groups (hydroxyl consuming) | 3.5° |

Sample A in accordance with the present invention showed hydrophilization of the surface and the comparison example (Sample B) did not show hydrophilization.

**Example 2, Illustrative examples:**

**Shampoo Compositions:**

| Ingredient | Wt. % Product I | Wt. % Product II |
|---|---|---|
| Water | Balance | Balance |
| Cetyl Alcohol | 4.18% | 4.18% |
| Stearyl Alcohol | 7.52% | 7.52% |
| Sodium laureth-3 sulfate (28% Active) | 10.00% | 10.00% |
| Lignin | 0.01% | 1.00% |

**Hair Conditioning:**

| Components | Wt% New Product I | Wt% New Product II |
|---|---|---|
| Behenyl trimethylammonium methosulfate | 2.97 | - |
| Stearamidopropyl dimethyl amine | - | 3.24 |
| Dicetyl dimethyl ammonium chloride | - | - |
| Cetyl alcohol | 1.01 | 4.25 |
| Stearyl alcohol | 2.53 | 2.93 |
| Benzyl alcohol | 0.4 | 0.4 |
| Deionized Water | Balance | Balance |
| L-glutamic acid | - | 1.04 |
| Preservative (Kathon CG) | 0 | 0 |
| Lignin | 0.01 | 1.00 |
| Aminosilicone *3 | 1.5 | 1.5 |
| Perfume | 0.5 | 0.5 |

**Hand Dishwashing:**

| Examples | Wt% Product I | Wt% Product II |
|---|---|---|
| Alkyl ethoxy sulfate AExS | 16 | 16 |
| Amine oxide | 5.0 | 5.0 |
| C9-11 EO8 | 5 | 5 |
| GLDA | 0.7 | 0.7 |
| Solvent | 1.3 | 1.3 |
| Polypropylene glycol (Mn=2000) | 0.5 | 0.5 |
| Sodium chloride | 0.8 | 0.8 |
| Lignin | 0.01 | 1.0 |
| Water | Balance | Balance |

**Granular laundry detergent compositions designed for front-loading automatic washing machines:**

| | Wt% Product I | Wt% Product II |
|---|---|---|
| Linear alkylbenzenesulfonate | 8 | 8 |
| C12-14 Alkylsulfate | 1 | 1 |
| AE7 | 2.2 | 2.2 |
| $C_{10-12}$ Dimethyl hydroxyethylammonium chloride | 0.75 | 0.75 |
| Crystalline layered silicate ($\delta$-$Na_2Si_2O_5$) | 4.1 | 4.1 |
| Zeolite A | 5 | 5 |
| Citric Acid | 3 | 3 |
| Sodium Carbonate | 15 | 15 |
| Silicate 2R ($SiO_2$:$Na_2O$ at ratio 2:1) | 0.08 | 0.08 |
| Soil release agent | 0.75 | 0.75 |
| Acrylic Acid/Maleic Acid Copolymer | 1.1 | 1.1 |
| Carboxymethylcellulose | 0.15 | 0.15 |
| Protease - Purafect® (84 mg active/g) | 0.2 | 0.2 |
| Amylase - Stainzyme Plus® (20 mg active/g) | 0.2 | 0.2 |
| Lipase - Lipex® (18.00 mg active/g) | 0.05 | 0.05 |
| Amylase - Natalase® (8.65 mg active/g) | 0.1 | 0.1 |
| TAED | 3.6 | 3.6 |
| Percarbonate | 13 | 13 |
| Na salt of Ethylenediamine-N,N'-disuccinic acid, (S,S) isomer (EDDS) | 0.2 | 0.2 |
| Hydroxyethane di phosphonate (HEDP) | 0.2 | 0.2 |
| $MgSO_4$ | 0.42 | 0.42 |
| Perfume | 0.5 | 0.5 |
| Suds suppressor agglomerate | 0.05 | 0.05 |

(continued)

| | Wt% Product I | Wt% Product II |
|---|---|---|
| Soap | 0.45 | 0.45 |
| Sulphonated zinc phthalocyanine (active) | 0.0007 | 0.0007 |
| S-ACMC | 0.01 | 0.01 |
| Lignin | 0.01 | 1.0 |
| Sulfate/ Water & Miscellaneous | Balance | Balance |

**Beauty Lotion/Cream:**

| | Wt% | Wt% |
|---|---|---|
| | **Product** I | **Product II** |
| Water | Balance | Balance |
| Glycerin | 7 | 7 |
| Disodium EDTA | 0.05 | 0.05 |
| Methylparaben | 0.1 | 0.1 |
| Sodium Dehydroacetate | 0.5 | 0.5 |
| Benzyl alcohol | 0.25 | 0.25 |
| GLW75CAP-MP (75% aq. $TiO_2$ dispersion)[1] | 0.5 | 0.5 |
| Palmitoyl-dipeptide[2] | 0.0001 | 0.0001 |
| N-acetyl glucosamine | 2 | 2 |
| Salicylic Acid | 1.5 | 1.5 |
| Isohexadecane | 3 | 3 |
| PPG 15 Stearyl Ether | 4 | 4 |
| Isopropyl Isostearate | 1.3 | 1.3 |
| Sucrose polyester | 0.7 | 0.7 |
| Phytosterol | 0.5 | 0.5 |
| Cetyl alcohol | 0.4 | 0.4 |
| Stearyl alcohol | 0.5 | 0.5 |
| Behenyl alcohol | 0.4 | 0.4 |
| PEG-100 stearate | 0.1 | 0.1 |
| Cetearyl glucoside | 0.1 | 0.1 |
| Polyacrylamide/C 13-14 isoparaffin/laureth-7 | 2 | 2 |
| Dimethicone/dimethiconol | 2 | 2 |
| Polymethylsilsequioxane | 0.25 | 0.25 |
| Lignin | 0.01 | 1.00 |

**Personal Care product containing skin lightening:**

| Component | Wt% Product I | Wt% Product II |
|---|---|---|
| Disodium EDTA | 0.100 | 0.100 |
| Phlorogine BG | 2.000 | 0 |
| deoxyArbutin | 0 | 2.000 |
| Niacinamide | 5.000 | 5.000 |
| Isohexadecane | 3.000 | 3.000 |
| Isopropyl isostearate | 1.330 | 1.330 |
| Sucrose polycottonseedate | 0.670 | 0.670 |
| Polymethylsilsesquioxane | 0.250 | 0.250 |
| Cetearyl glucoside + cetearyl alcohol | 0.200 | 0.200 |
| Behenyl alcohol | 0.400 | 0.400 |
| Ethylparaben | 0.200 | 0.200 |
| Propylparaben | 0.100 | 0.100 |
| Cetyl alcohol | 0.320 | 0.320 |
| Stearyl alcohol | 0.480 | 0.480 |
| Tocopheryl acetate | 0.500 | 0.500 |
| PEG-100 stearate | 0.100 | 0.100 |
| Glycerin | 7.000 | 7.000 |
| Titanium dioxide | 0.604 | 0.604 |
| Polyacrylamide + C13-14 isoparaffin + laureth-7 | 2.000 | 2.000 |
| Panthenol | 1.000 | 1.000 |
| Benzyl alcohol | 0.400 | 0.400 |
| Dimethicone + dimethiconol | 2.000 | 2.000 |
| Lignin | 0.010 | 1.000 |
| Water (to 100g) | Balance | Balance |

**Automatic Dishwashing Cleaning composition:**

| | Powder (wt% based on 19 g portion) | Powder (wt% based on 19 g portion) |
|---|---|---|
| STPP | 34-38 | 34-38 |
| Alcosperse[1] | 7-12 | 7-12 |
| SLF-18 Polytergent[2] | 1-2 | 1-2 |
| Esterified substituted benzene sulfonate[3] | 0.1-6.0 | 0.1-6.0 |
| Polymer[4] | 0.2-6.0 | 0.2-6.0 |
| Sodium perborate monohydrate | 2-6 | 2-6 |

(continued)

| | Powder (wt% based on 19 g portion) | Powder (wt% based on 19 g portion) |
|---|---|---|
| Carbonate | 20-30 | 20-30 |
| 2.0r silicate | 5-9 | 5-9 |
| Sodium disilicate | 0-3 | 0-3 |
| Enzyme system[5] | 0.1-5.0 | 0.1-5.0 |
| Pentaamine cobalt(III)chloride dichloride salt | 10-15 | 10-15 |
| TAED | 0-3 | 0-3 |
| Perfume, dyes, water and other components | Balance to 100% | Balance to 100% |
| | Liquid (wt% based on 1.9 g portion) | Liquid (wt% based on 1.9 g portion) |
| Dipropylene Glycol | 35-45 | 35-45 |
| SLF-19 Polytergent[2] | 40-50 | 40-50 |
| Neodol ® C11EO9 | 1-3 | 1-3 |
| Lignin | 0.01 | 1.0 |
| Dyes, water and other components | Balance | Balance |

[1] such as Alcosperse® 246 or 247, a sulfonated copolymer of acrylic acid from Alco Chemical Co.
[2] linear alcohol ethoxylate from Olin Corporation
[3] such as those described above
[4] a sulfonated polymer such as those described above
[5] one or more enzymes such as protease, mannaway, natalase, lipase and mixture thereof.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A consumer goods product comprising a consumer goods product ingredient and a non-cross linked functionalised lignin oligomer,
wherein the lignin oligomer:

(a) has a number average molecular weight ($\overline{M}_n$) in the range of from 800 Da to 1,800 Da;
(b) comprises the functional group:

lignin_backbone-O-L-COOH

wherein: 'lignin_backbone' is the lignin structural backbone; and
L is a linker comprising an -OH moiety;
(c) has a hydroxyl content of at least 3 mmol/g; and
(d) has a functionalisation content of from 0.2 mmol/g to 2.3 mmol/g.

2. A consumer goods product according to claim 1, wherein L is a linker having a chemical structure:

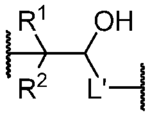

and / or

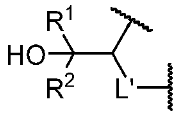

wherein R1 and R2 are independently chosen from a group consisting of H and linear or branched, saturated or unsaturated, substituted or unsubstituted $C_1$ to $C_{18}$ alkyl; and wherein L' is a linking motif chosen from linear or branched, saturated or unsaturated, substituted or unsubstituted $C_1$-$C_{18}$ alkyl.

**3.** A consumer goods product according to any preceding claim, wherein the lignin oligomer has a functionalisation content of from 0.6 mmol/g to 1.0 mmol/g.

**4.** A consumer goods product according to any preceding claim, wherein the lignin oligomer has a hydroxyl content of from 3 mmol/g to 5.7 mmol/g.

**5.** A consumer goods product according to any preceding claim, wherein the lignin oligomer comprises less than 1wt% sulphur content.

**6.** A consumer goods product according to any preceding claim, wherein the lignin oligomer has a molar ratio of aromatic hydroxyl content to aliphatic hydroxyl content in the range of from 1:1 to 1.5:1.

**7.** A consumer goods product according to any preceding claim, wherein the lignin oligomer has a weight average molecular weight ($\overline{M}_w$) in the range of from 800 Da to 5000 Da.

**8.** A consumer goods product according to any preceding claim, wherein the lignin oligomer has a number average molecular weight ($\overline{M}_n$) in the range of from 800 Da to 1200 Da.

**9.** A consumer goods product according to any preceding claim, wherein the lignin oligomer is essentially free of sulphur.

**10.** A consumer goods product according to any preceding claim, wherein the lignin oligomer has an ester content in the range of from 0.0 mmol/g to 0.1 mmol/g.

**11.** A consumer goods product according to any preceding claim, wherein the lignin oligomer is derived from corn, sugar cane, wheat and any combination thereof.

**12.** A consumer goods product according to any preceding claim, wherein the consumer goods product comprises an emollient and/or humectant.

**13.** A consumer goods product according to any preceding claim, wherein the consumer goods product comprises an emulsifier, and wherein the lignin oligomer is in the form of an emulsion.

**14.** A consumer goods product according to any claims 1-13, wherein the product is a skin treatment composition.

**15.** A consumer goods product according to any of claims 1-13, wherein the product is a hair treatment composition.

**16.** A consumer goods product according to any of claims 1-13, wherein the product is an oral care composition.

**17.** A consumer goods product according to any of claims 1-13, wherein the product is an antiseptic cream.

**18.** A consumer goods product according to any of claims 1-13, wherein the product is shoe polish.

**19.** A consumer goods product according to any of claims 1-13, wherein the product is a detergent composition.

**20.** A consumer goods product according to any preceding claim, wherein the consumer goods product comprises chitin and/or chitin derivatives.

Europäisches Patentamt
European Patent Office
Office européen des brevets

# EUROPEAN SEARCH REPORT

Application Number
EP 15 17 3588

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2008/125544 A1 (YAO KENJI [JP]) 29 May 2008 (2008-05-29)<br>* the whole document *<br>----- | 1 | INV.<br>A61K8/72<br>C07G1/00<br>C08H7/00<br>C08L97/00<br>A61Q5/02<br>A61Q5/12<br>A61Q19/00<br>C11D3/382<br>C09G1/00 |
| A | US 2003/156970 A1 (OBERKOFLER JORG [AT] ET AL) 21 August 2003 (2003-08-21)<br>* the whole document *<br>----- | 1 | |
| A | DATABASE GNPD [Online]<br>MINTEL;<br>March 2009 (2009-03),<br>"Eye contour cream",<br>XP002751746,<br>Database accession no. 1102156<br>* the whole document *<br>----- | 1 | |
| A | DATABASE GNPD [Online]<br>MINTEL;<br>April 2012 (2012-04),<br>"Aloe vera shower gel",<br>XP2751693,<br>Database accession no. 1765683<br>* the whole document *<br>----- | 1 | TECHNICAL FIELDS SEARCHED (IPC) |
| A | UGARTONDO V ET AL: "Comparative antioxidant and cytotoxic effects of lignins from different sources", BIORESOURCE TECHNOLOGY, ELSEVIER BV, GB, vol. 99, no. 14, 1 September 2008 (2008-09-01), pages 6683-6687, XP022679252, ISSN: 0960-8524, DOI: 10.1016/J.BIORTECH.2007.11.038 [retrieved on 2008-01-09]<br>* the whole document *<br>-----<br>-/-- | 1 | A61K<br>C07G<br>C08H<br>C08L<br>A61Q<br>C11D<br>C09G |

1

The present search report has been drawn up for all claims

| Place of search | Date of completion of the search | Examiner |
|---|---|---|
| Munich | 16 December 2015 | Donovan-Beermann, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 15 17 3588

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XUEJUN PAN ET AL: "Organosolv Ethanol Lignin from Hybrid Poplar as a Radical Scavenger: Relationship between Lignin Structure, Extraction Conditions, and Antioxidant Activity", JOURNAL OF AGRICULTURAL AND FOOD CHEMISTRY, AMERICAN CHEMICAL SOCIETY, US, vol. 54, no. 16, 9 August 2006 (2006-08-09), pages 5806-5813, XP008148495, ISSN: 0021-8561, DOI: 10.1021/JF0605392 [retrieved on 2006-07-07] * the whole document * ----- | 1 | |
| A | US 2003/139319 A1 (SCHEIBEL JEFFREY JOHN [US]) 24 July 2003 (2003-07-24) * the whole document * ----- | 1 | |
| A | US 2 352 021 A (ERNST SCHUBERT ET AL) 20 June 1944 (1944-06-20) * the whole document * ----- | 1 | TECHNICAL FIELDS SEARCHED (IPC) |
| A | LORA J H ET AL: "Recent industrial applications of lignin: a sustainable alternative to nonrenewable materials", JOURNAL OF POLYMERS AND THE ENVIRONMENT, SPRINGER NEW YORK LLC, US, vol. 10, no. 1/2, 1 January 2002 (2002-01-01), pages 39-48, XP002493248, ISSN: 1566-2543, DOI: 10.1023/A:1021070006895 * the whole document * ----- | 1 | |

The present search report has been drawn up for all claims

1

| Place of search | Date of completion of the search | Examiner |
|---|---|---|
| Munich | 16 December 2015 | Donovan-Beermann, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 17 3588

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2015

| Patent document cited in search report | Publication date | Patent family member(s) | Publication date |
|---|---|---|---|
| US 2008125544 A1 | 29-05-2008 | EP 1927616 A2 | 04-06-2008 |
| | | JP 4135760 B2 | 20-08-2008 |
| | | JP 2008133368 A | 12-06-2008 |
| | | US 2008125544 A1 | 29-05-2008 |
| US 2003156970 A1 | 21-08-2003 | AT 257128 T | 15-01-2004 |
| | | AU 4831501 A | 24-09-2001 |
| | | CA 2403364 A1 | 16-09-2002 |
| | | CN 1423622 A | 11-06-2003 |
| | | EP 1272433 A2 | 08-01-2003 |
| | | HK 1055104 A1 | 08-09-2006 |
| | | JP 4124594 B2 | 23-07-2008 |
| | | JP 2003527234 A | 16-09-2003 |
| | | US 2003156970 A1 | 21-08-2003 |
| | | WO 0168530 A2 | 20-09-2001 |
| US 2003139319 A1 | 24-07-2003 | BR 0306943 A | 14-12-2004 |
| | | CA 2471591 A1 | 31-07-2003 |
| | | CN 1617878 A | 18-05-2005 |
| | | EP 1465904 A1 | 13-10-2004 |
| | | JP 2005522529 A | 28-07-2005 |
| | | MX PA04006938 A | 06-12-2004 |
| | | US 2003139319 A1 | 24-07-2003 |
| | | WO 03062254 A1 | 31-07-2003 |
| US 2352021 A | 20-06-1944 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **J. ASIKKALA ; T. TAMMINEN ; D. S. ARGYROPOULOS.** *J. Agric. Food Chem.,* 2012, vol. 60, 8968-8973
- **A. GRANATA ; D. S. ARGYROPOULOS.** *J. Agric. Food Chem.,* 1995, vol. 43, 1538-1544
- **DIETZ, T.** Basic properties of cosmetic oils and their relevance to emulsion preparations. *SOFW-Joumal,* July 1999, 1-7